# EUROPEAN PATENT APPLICATION

(11) **EP 4 563 564 A1**
(43) Date of publication of application: **04.06.2025**
(21) Application number: 23846601.5
(22) Date of filing: 27.07.2023
(51) Int. Cl.: C07C 17/20, C07C 19/10

(54) **METHOD FOR PRODUCING 1,2,3,4-TETRACHLORO-1,1,2,3,4,4-HEXAFLUOROBUTANE**

(30) Priority: 28.07.2022 JP 2022120820
(71) Applicant: Kanto Denka Kogyo Co., Ltd., Tokyo 100-0005 (JP)
(72) Inventor: KIKUCHI, Sho, Kurashiki-shi, Okayama 712-8533 (JP); OKUYAMA, Yohei, Kurashiki-shi, Okayama 712-8533 (JP); KISHI, Kazuki, Kurashiki-shi, Okayama 712-8533 (JP); ASAHINA, Kazuma, Kurashiki-shi, Okayama 712-8533 (JP); TANIDA, Tatsumi, Shibukawa-shi, Gunma 377-8513 (JP)
(74) Representative: Pfenning, Meinig & Partner mbB
(86) International application number: PCT/JP2023/027484
(87) International publication number: WO 2024/024871

(57) **Abstract**

The present invention aims at that in a series of processes of producing 1,2,3,4-tetrachlorohexafluorobutane from 1,3-butadiene, by-products can be converted to a target substance by a relatively simple operation, raising the production efficiency as the whole process. There is provided a method for producing 1,2,3,4-tetrachloro-1,1,2,3,4,4-hexafluorobutane comprising step (a) of reacting chlorofluorobutane represented by the formula (1): C₄ClₙF₁₀₋ₙ (1), wherein n is an integer satisfying 5 ≤ n ≤ 7, with chlorine monofluoride (ClF) to thereby produce 1,2,3,4-tetrachloro-1,1,2,3,4,4-hexafluorobutane.

## Description

### TECHNICAL FIELD

The present invention relates to a method for producing 1,2,3,4-tetrachloro-1,1,2,3,4,4-hexafluorobutane (hereinafter, referred to simply as 1,2,3,4-tetrachlorohexafluorobutane) by forming 1,2,3,4-tetrachlorobutane by chlorinating 1,3-butadiene, and replacing hydrogen atoms of the 1,2,3,4-tetrachlorobutane by fluorine atoms. The present invention relates particularly to a method for producing 1,2,3,4-tetrachlorohexafluorobutane, the method comprising a step of forming and recovering 1,2,3,4-tetrachlorohexafluorobutane by reacting the following by-products:

C₄ClₙF₁₀₋ₙ (1)

wherein n is 5 ≤ n ≤ 7,
formed in the reaction process, with chlorine monofluoride (ClF). The present invention relates more particularly to a method for producing 1,2,3,4-tetrachlorohexafluorobutane, which is useful as a synthesis raw material of hexafluoro-1,3-butadiene, which is paid attention to as a semiconductor etching gas or the like.

### BACKGROUND ART

1,2,3,4-Tetrachlorohexafluorobutane is an important compound as a synthesis raw material or the like of hexafluoro-1,3-butadiene to be used as an etching gas to be used in microfabrication for semiconductors. As a method for producing the 1,2,3,4-tetrachlorohexafluorobutane, methods described in the following Patent Literatures are conventionally known.
(1) In Japanese Patent Laid-Open No. 2006-342059 (PTL 1), there is described a method for producing 1,2,3,4-tetrachlorohexafluorobutane by reacting a compound represented by CClX¹X²-CClX³-CClX⁴-CClX⁵X⁶ (X is a hydrogen atom or a fluorine atom) with fluorine in a liquid phase.
(2) In Japanese Patent No. 5274449 (PTL 2), there is described a method for producing 1,2,3,4-tetrachlorohexafluorobutane, wherein the method includes regulating the amount of hydrogen fluoride in a solvent to be used for reaction of 1,2,3,4-tetrachlorobutane with fluorine in the amount range of 10 to 60% by mass.
(3) In Japanese Patent No. 5430567 (PTL 3), there is described a method for producing 1,2,3,4-tetrachlorohexafluorobutane, wherein the method includes a step of synthesizing a crude 1,2,3,4-tetrachlorohexafluorobutane by reacting 1,2,3,4-tetrachlorobutane with fluorine gas and introducing the reaction product to a first distillation column having the number of theoretical stages of 15 or more to obtain a distillate containing mainly 1,2,3,4-tetrachlorohexafluorobutane from the column top of the first distillation column, and a step of introducing the distillate to a second distillation column having the number of theoretical stages of 25 or more to obtain a refined 1,2,3,4-tetrachlorohexafluorobutane from the column bottom of the second distillation column.

However, the methods described in these Patent Literatures have the following problems.
In the method of (1), when a reproducing experiment of the fluorination reaction of 1,2,3,4-tetrachlorobutane is carried out, in addition to 1,2,3,4-tetrachlorohexafluorobutane being the target substance, as main impurities, several kinds of by-products containing 1,1,2,3,4-pentachloro-1,2,3,4,4-pentafluorobutane (hereinafter, referred to simply as 1,1,2,3,4-pentachloropentafluorobutane) are generated.
In the method of (2), whereas addition of HF to a reaction solvent enables reaction in a low-temperature region, thereby bringing about the effect of suppressing the generation of low-boiling components mainly by cleavage of C-C bonds and the excess progress of fluorination, the generation of pentachloropentafluorobutane as by-products does not come to be sufficiently suppressed.
In the method of (3), although high-boiling components containing pentachloropentafluorobutane are separated by distillation operation, and a high-purity 1,2,3,4-tetrachlorohexafluorobutane is finally obtained, generally in distillation operation, where the product purity is given priority, the product recovery ratio per unit time lowers. Further, it is difficult to completely recover, as a product, a target component contained in a column bottom liquid containing mainly high-boiling components.

That is, since there are such a problem that the reaction selectivity of 1,2,3,4-tetrachlorohexafluorobutane lowers due to by-production of pentachloropentafluorobutane in a fluorination reaction step, and such a problem that when high-boiling components containing mainly pentachloropentafluorobutane are separated and removed in a distillation refinement step, a column bottom liquid containing mainly pentachloropentafluorobutane, and containing a certain amount of 1,2,3,4-tetrachlorohexafluorobutane is treated as industrial waste, the production efficiency of 1,2,3,4-tetrachlorohexafluorobutane lowers.

Further, although there are cases where use of additives (catalyst, HF and the like) improves the reaction efficiency, since after the finish of the reaction, the operation of separation of the additives (catalyst, HF and the like) becomes necessary, the improvement does not always lead to an improvement in the production efficiency as the whole production process. Therefore, also on this account, the conventional method of producing 1,2,3,4-tetrachlorohexafluorobutane by reacting 1,2,3,4-tetrachlorobutane with fluorine leaves a problem in that a target substance needs to be produced industrially more efficiently.

### CITATION LIST

### PATENT LITERATURE

PTL 1: Japanese Patent Laid-Open No. 2006-342059
PTL 2: Japanese Patent No. 5274449
PTL 3: Japanese Patent No. 5430567

### SUMMARY OF INVENTION

### TECHNICAL PROBLEM

As described above, there is such a problem that in the fluorination step in a series of processes for producing 1,2,3,4-tetrachlorohexafluorobutane from 1,3-butadiene, by-products represented by the following formula (1):

C₄ClₙF₁₀₋ₙ (1)

wherein n is an integer satisfying 5 ≤ n ≤ 7,
including pentachloropentafluorobutane, are formed. That is, since there are such a problem that the reaction selectivity of 1,2,3,4-tetrachlorohexafluorobutane lowers due to by-production of compounds of the formula (1) in the fluorination reaction step, and such a problem that when high-boiling components containing mainly compounds of the formula (1) are separated and removed in a distillation refinement step, a column bottom liquid containing mainly the compounds of the formula (1) containing a certain amount of 1,2,3,4-tetrachlorohexafluorobutane is treated as industrial waste, there arises such a problem that the production efficiency of 1,2,3,4-tetrachlorohexafluorobutane lowers.

Then, the problem of the present invention is, in a series of processes for producing 1,2,3,4-tetrachlorohexafluorobutane from 1,3-butadiene, to enable by-products to be converted to a target substance by a relatively simple operation, raising the production efficiency as the whole process.

### SOLUTION TO PROBLEM

The present invention provides the following.
[1] A method for producing 1,2,3,4-tetrachloro-1,1,2,3,4,4-hexafluorobutane, comprising:
   step (a) of reacting chlorofluorobutane represented by the following formula (1):

   C₄ClₙF₁₀₋ₙ (1)

   wherein n is an integer satisfying 5 ≤ n ≤ 7,
   with chlorine monofluoride (ClF) to thereby produce 1,2,3,4-tetrachloro-1,1,2,3,4,4-hexafluorobutane.
[2] The method according to [1], further comprising, before the step (a), step (b) of reacting 1,2,3,4-tetrachlorobutane with fluorine gas (F₂) to thereby produce a reaction liquid including chlorofluorobutane represented by the formula (1).
[3] The method according to [2], wherein in the step (b), 1,2,3,4-tetrachloro-1,1,2,3,4,4-hexafluorobutane is used as a solvent.
[4] The method according to any one of [1] to [3], wherein the chlorofluorobutane represented by the formula (1) includes 1,1,2,3,4-pentachloro-1,2,3,4,4-pentafluorobutane.
[5] A method for producing 1,2,3,4-tetrachloro-1,1,2,3,4,4-hexafluorobutane, comprising:
   step (B) of reacting a reaction liquid including 1,2,3,4-tetrachlorobutane with fluorine gas (F₂) to thereby produce a reaction liquid including 1,2,3,4-tetrachloro-1,1,2,3,4,4-hexafluorobutane and chlorofluorobutane represented by the following formula (1):

      C₄ClₙF₁₀₋ₙ (1)

      wherein n is an integer satisfying 5 ≤ n ≤ 7; and
   step (C) of reacting the reaction liquid obtained by step (B) with chlorine monofluoride (ClF) to thereby convert the chlorofluorobutane represented by the formula (1) in the reaction liquid obtained by step (B) to 1,2,3,4-tetrachloro-1,1,2,3,4,4-hexafluorobutane.
[6] The method according to [5], wherein the chlorofluorobutane represented by the formula (1) includes 1,1,2,3,4-pentachloro-1,2,3,4,4-pentafluorobutane.
[7] The method according to [5] or [6], further comprising step (A) of reacting 1,3-butadiene with chlorine gas (Cl₂) to thereby produce a reaction liquid including 1,2,3,4-tetrachlorobutane, wherein the reaction liquid obtained by step (A) is used as a raw material of step (B).
[8] The method according to any one of [1] to [4], wherein in the step (a), the reaction is carried out in the presence of hydrogen fluoride (HF).
[9] The method according to any one of [5] to [7], wherein in the step (C), the reaction is carried out in the presence of hydrogen fluoride (HF).

### ADVANTAGEOUS EFFECTS OF INVENTION

According to the present invention, advantageous effects are attained in two aspects in which organic waste liquids (high-boiling components of by-products and the like) generated in the 1,2,3,4-tetrachlorohexafluorobutane production step are converted to the valuable material, 1,2,3,4-tetrachlorohexafluorobutane, and the 1,2,3,4-tetrachlorohexafluorobutane further becomes enabled to be converted to hexafluoro-1,3-butadiene, salable as a product (merit of productivity improvement), and the amount of industrial wastes, particularly waste liquids to be treated as fluorine/chlorine-containing organic waste liquids, can be reduced (merit of the environmental load reduction). Chlorine monofluoride (ClF) to be reacted with chlorofluorobutane represented by C₄ClₙF₁₀₋ₙ (wherein n is an integer satisfying 5 ≤ n ≤ 7) containing mainly 1,1,2,3,4-pentachloropentafluorobutane can be massively and stably generated by reacting a gas containing ClF₃ or F₂ with a gas containing Cl₂ by the method described in Japanese Patent No. 6246449, and can be supplied safely for a long time.

### DESCRIPTION OF EMBODIMENTS

### (Action)

The present inventors have found that in a process of producing 1,2,3,4-tetrachlorohexafluorobutane, by making chlorine monofluoride (ClF) to act on a crude reaction liquid containing by-products containing chlorofluorobutane represented by the following formula (1):

C₄ClₙF₁₀₋ₙ (1)

wherein n is an integer satisfying 5 ≤ n ≤ 7, particularly, an integer satisfying 5 ≤ n ≤ 6,
or on a solution containing the by-products concentrated to high concentrations by a refining operation such as distillation, the reaction by-products represented by the formula (1) can be converted to 1,2,3,4-tetrachlorohexafluorobutane. In the present invention, by utilizing this novelly found reaction, instead of suppressing side reactions by using an additive or catalyst, the by-products are successfully efficiently converted to a target substance, thereby materializing reduction in by-products without needing a removal operation of the additive or catalyst after the reaction, and materializing the process as a whole high in the production efficiency.

### (Reaction of chlorofluorobutane of the formula (1) with chlorine monofluoride)

In the present invention, a method for producing 1,2,3,4-tetrachlorohexafluorobutane comprises a step of reacting chlorofluorobutane represented by the following formula (1):

C₄ClₙF₁₀₋ₙ (1)

wherein n is an integer satisfying 5 ≤ n ≤ 7, particularly, an integer satisfying 5 ≤ n ≤ 6, with chlorine monofluoride (ClF) to thereby produce 1,2,3,4-tetrachlorohexafluorobutane. Examples of the chlorofluorobutane of the formula (1) include the following. 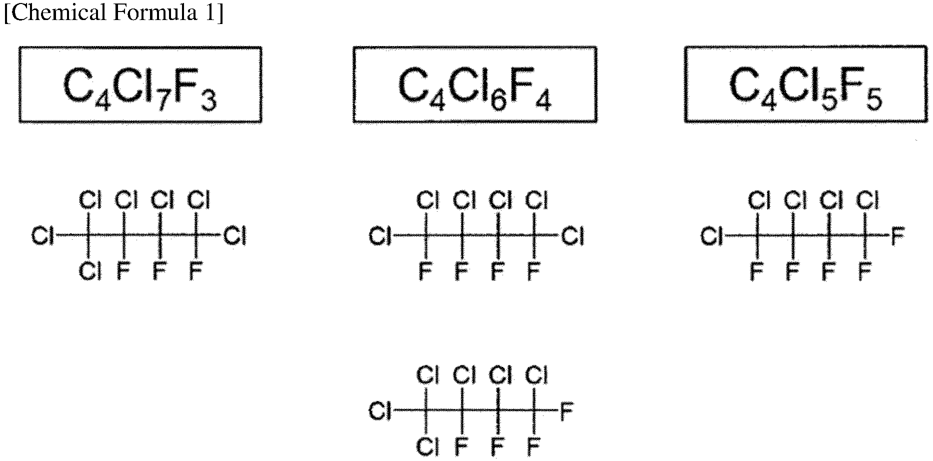 Among the above by-products, by-products formed at relatively high selectivities include 1,1,2,3,4-pentachloropentafluorobutane, and since such by-products can be converted to a target substance, the present invention is very useful.

Preferable reaction conditions in the reaction of chlorofluorobutane of the formula (1) with chlorine monofluoride are, for example, as follows.

### Reaction temperature:

When the reaction temperature is low, the reactivity of the chlorine monofluoride lowers and the chlorine monofluoride cannot be utilized efficiently for the reaction. On the other hand, when the reaction temperature is high, since more energy is needed, the case is not economical. For these reasons, the reaction temperature is preferably 50°C to 180°C and more preferably 80°C to 150°C.

### Reaction pressure:

The ClF gas is high in the reactivity and in order to carry out the reaction stably and continuously for a long time, a high pressure is not preferable. Hence, the pressure in a reactor is set, in gauge pressure, preferably at -0.02 to 0.20 MPaG and more preferably at 0 to 0.10 MPaG. Hereinafter, the gauge pressure is represented as "MPaG".

Constitution of a desirable reaction apparatus:
(i) In the case where the reaction is carried out in a liquid phase, it is desirable to use, as a solvent, 1,2,3,4-tetrachlorohexafluorobutane (boiling point: 134°C), which is the target substance. A first reason is because since the target substance is used as a solvent, a step of separating the solvent after the reaction can be omitted. Further, a second reason is because also in the reaction of 1,2,3,4-tetrachlorobutane with fluorine in the preceding step described later, the 1,2,3,4-tetrachlorohexafluorobutane being the target substance is similarly used as a reaction solvent.
(ii) Materials of the reaction apparatus including a reaction vessel and piping include alloys such as stainless steel, Monel metal, inconel and Hastelloy, and glass, nickel, polytetrafluoroethylene (PTFE) and perfluoroalkoxyalkanes (PFA), but in the case where the reaction is carried out in the atmospheric pressure, a glass-made reaction vessel, or a PTFE-made or PFA-made reactor can be used.
(iii) As a method of introducing ClF, a usual method such as a single pipe type, or an aeration plate type (sintered metallic filter or PTFE filter) is used, and by reducing the bubble size, the contact efficiency between gas and liquid can be raised, whereby the utilization efficiency of ClF can be raised. Further, by combining usual methods, such as a method in which a plurality of reactors are connected in series and the chlorofluorobutane and ClF gas are brought into contact, and a part or the whole of unreacted ClF gas discharged from a preceding-stage reactor is introduced to a succeeding-stage reactor, and a method in which the reactor is installed with a stirring device, the utilization efficiency of ClF can be raised.
(iv) The reaction may be carried out in a batch system or a continuous system. The batch system can make the reaction to be simply carried out, and the continuous system can make the reaction to be carried out efficiently. In the case where the reaction is carried out in the continuous system, a plurality of reactors are connected in series, and by introducing ClF to the preceding stage-side reactor and introducing the chlorofluorobutane to the succeeding stage-side reactor, unreacted ClF discharged from the preceding stage-side reactor can be effectively utilized in the succeeding stage-side reactor.
(v) A reaction liquid (crude 1,2,3,4-tetrachlorohexafluorobutane) obtained by the reaction of chlorofluorobutane of the formula (1) with chlorine monofluoride can be highly purified by a usual refining operation such as distillation.
(vi) ClF gas concentration:
   The ClF gas may be simple ClF gas, but may be introduced as a diluted mixed gas diluted with an inert gas such as nitrogen. In the case where the concentration of the ClF gas is high, and the flow rate thereof is high, the reaction is caused locally excessively, and trichloroheptafluorobutane is formed, causing a reduction in the reaction selectivity. Then, also in order to continue the reaction stably and continuously for a long time, it is favorable to regulate the balance between the concentration of the ClF gas and the flow rate to control the reactivity. Then, the case where the concentration of the ClF gas is low, the production efficiency lowers, which is industrially disadvantageous. Hence, it is favorable that the concentration of the ClF gas is regulated, though needing to take a balance with the flow rate into consideration, preferably at 20 to 100% by volume and more preferably at 60 to 100% by volume. The ClF gas is introduced at a flow rate of preferably 1 to 3,000 L/h and more preferably 1 to 1,500 L/h. In particular, in the case where the reaction is carried out in an industrial scale, depending on the reactor size, the flow rate needs to be a high flow rate of 500 L/h or higher. Then, since along with the progress of the reaction, the concentration of 1,2,3,4-tetrachlorohexafluorobutane in the reaction liquid increases and the condition becomes that of easily forming excess-reaction products, along with the progress of the reaction, the flow rate and the concentration of the ClF gas to be introduced may suitably be regulated.
(vii) Other reaction conditions:
   The progress of the reaction can be checked by using usual analyzing devices such as gas chromatography.
   1,1,2,3,4-Pentachloropentafluorobutane may be allowed to react in a form of a simple substance, but may be allowed to react in a solvent.
   By installing the succeeding stage of the reactors with a condenser for reliquefying vapor of the reaction liquid, the loss of the reaction liquid can be reduced and the production efficiency can be raised. Then, by installing the succeeding stage of the reactors with an apparatus for liquefying and recovering vapor of 1,2,3,4-tetrachlorohexafluorobutane by utilizing a difference in boiling point between the chlorofluorobutane being a raw material and 1,2,3,4-tetrachlorohexafluorobutane, the production efficiency can be raised.
(viii) Additive:
   By adding hydrogen fluoride (HF), the reactivity of the chlorine monofluoride (ClF) is improved and the reaction can be carried out efficiently. Although making HF present in the reaction liquid is vital, since the reaction liquid is heated up to a temperature of the boiling point of HF or higher, HF continually evaporates and migrates to a gas phase. Hence, by connecting a cooling condenser to a gas outlet port of the reactor, and condensing HF vapor and again returning HF to the reaction liquid, the effect can be attained efficiently. Then, since when HF evaporates and the HF concentration in the reaction liquid lowers, the effect deteriorates, it is preferable to introduce HF in the course of the reaction. HF may be introduced continuously at the same time with ClF, and in this case, it is favorable that the amount ratio of HF to be introduced to ClF is regulated preferably at 0.003 to 0.08 mol times and more preferably at 0.003 to 0.04 mol times.

A preferable mode carries out, for example, before the reaction of chlorofluorobutane of the formula (1) with chlorine monofluoride, a step of reacting 1,2,3,4-tetrachlorobutane with fluorine gas (F₂) to thereby produce a reaction liquid containing chlorofluorobutane represented by the formula (1). The chlorofluorobutane represented by the formula (1) is formed as by-products when 1,2,3,4-tetrachlorohexafluorobutane is produced by reacting 1,2,3,4-tetrachlorobutane with fluorine gas (F₂). The reaction with chlorine monofluoride in the following step may be carried out after chlorofluorobutane of the formula (1) is isolated or concentrated from the reaction liquid obtained by the reaction with fluorine gas, by an operation such as distillation, or may be carried out by using the reaction liquid obtained by the reaction with fluorine gas, as it is. In the case of the former (isolation or concentration), 1,2,3,4-tetrachlorohexafluorobutane can be obtained efficiently; in the case of the latter, the formation of an excess-reaction product (C₄Cl₃F₇) reduces the yield, but a step of isolating or concentrating chlorofluorobutane of the formula (1) by distillation can be omitted.

Preferable reaction conditions in the reaction of 1,2,3,4-tetrachlorobutane with fluorine gas (F₂) are, for example, as follows.

### Solvent:

Usable solvents are, in addition to 1,2,3,4-tetrachlorohexafluorobutane, chlorocarbons exemplified by tetrachloromethane and hexachloroethane, and chlorofluorocarbons exemplified by 1,1,2-trichloro-1,2,2-trifluoroethane and 1,1,2,2-tetrachloro-1,2-difluoroethane.

### Reaction temperature:

In the case where the reaction temperature is low, the utilization efficiency of the fluorine gas lowers; in the case where the reaction temperature is high, decomposition reaction involving cleavage of C-C bonds may possibly occur, causing the lowering of the reaction yield. For these reasons, it is favorable that the reaction temperature is set preferably at 10 to 100°C and more preferably at 20 to 70°C.

### Reaction pressure:

Fluorine gas is high in the reactivity and in order to continue the reaction stably and continuously for a long time, a high pressure is not preferable. Hence, it is favorable that the pressure in a reactor is set, in gauge pressure, preferably at -0.02 to 0.20 MPaG and more preferably at 0 to 0.10 MPaG.

### Fluorine gas concentration:

The fluorine gas to be used for the reaction may be simple fluorine gas, but may be introduced as a diluted mixed gas diluted with an inert gas such as nitrogen. In the case where the concentration of the fluorine gas is high, decomposition reaction involving cleavage of C-C bonds may possibly occur, causing the lowering of the reaction yield. Then, also in order to continue the reaction stably and continuously for a long time, it is favorable that the concentration of the fluorine gas is regulated and the reactivity is controlled. Then, in the case where the concentration of the fluorine gas is low, the production efficiency lowers, which is industrially disadvantageous. Hence, it is favorable that the concentration of the fluorine gas is regulated preferably at 20 to 80% by volume and more preferably at 40 to 70% by volume.

Constitution of a desirable reaction apparatus:
In the case where the reaction is carried out in a liquid phase, it is desirable to use, as a solvent, 1,2,3,4-tetrachlorohexafluorobutane (boiling point: 134°C), which is the target substance. The reason is because since the target substance is used as a solvent, a step of separating the solvent after the reaction can be omitted.

Materials of the reaction apparatus including a reaction vessel and piping include alloys such as stainless steel, Monel metal, inconel and Hastelloy, and nickel. Only in the case where the concentration of the fluorine gas is regulated to 20% or lower and the reaction is carried out at the atmospheric pressure, a PTFE-made reactor can also be used.

With regard to a method of introducing fluorine gas, by combining usual methods, such as a method in which a plurality of reactors are connected in series, and 1,2,3,4-tetrachlorobutane and/or reaction intermediates in which hydrogen atoms of the 1,2,3,4-tetrachlorobutane are partially replaced by fluorine atoms are brought into contact with fluorine gas and a part or the whole of unreacted fluorine gas discharged from a preceding-stage reactor is introduced to a succeeding-stage reactor, and a method in which the reactor is installed with a stirring device, the utilization efficiency of the fluorine gas can be raised.

A crude 1,2,3,4-tetrachlorohexafluorobutane obtained by the reaction of 1,2,3,4-tetrachlorobutane with fluorine gas can be highly purified by a usual refining operation such as distillation.

An applicable example for the reaction of chlorofluorobutane of the formula (1) with chlorine monofluoride in the method (hereinafter, referred to as butadiene method) for producing 1,2,3,4-tetrachlorohexafluorobutane through 1,2,3,4-tetrachlorobutane by using 1,3-butadiene as a starting raw material)

Since the chlorofluorobutane of the formula (1) is by-products in the 1,2,3,4-tetrachlorohexafluorobutane production method (butadiene method) and the above-mentioned reaction of the chlorofluorobutane of the formula (1) with chlorine monofluoride can convert the by-products to the target substance, the reaction is useful. Specifically, by an integrated process comprising the following series of steps, 1,2,3,4-tetrachlorohexafluorobutane can be produced efficiently.

The series of steps include:
step (A) of reacting 1,3-butadiene with chlorine gas (Cl₂) to produce a reaction liquid containing 1,2,3,4-tetrachlorobutane;
step (B) of reacting the reaction liquid containing 1,2,3,4-tetrachlorobutane obtained by step (A) with fluorine gas (F₂) to produce a reaction liquid containing 1,2,3,4-tetrachlorohexafluorobutane and chlorofluorobutane represented by the following formula (1):

   C₄ClₙF₁₀₋ₙ (1)

   wherein n is an integer satisfying 5 ≤ n ≤ 7; and
step (C) of reacting the reaction liquid obtained by step (B) with chlorine monofluoride (ClF) to convert the chlorofluorobutane represented by the formula (1) to 1,2,3,4-tetrachlorohexafluorobutane.

With regard to step (A), it is well-known in the art that a reaction liquid containing 1,2,3,4-tetrachlorobutane is obtained by reacting 1,3-butadiene with chlorine gas (Cl₂), but in the series of processes of the present invention, preferable reaction conditions are as follows.

### Reaction temperature:

Preferably -20 to 40°C, more preferably -20 to 20°C

### Reaction pressure:

Preferably 0 to 1.5 MPaG, more preferably 0 to 0.3 MPaG

### Reaction solvent:

Specific examples of preferable solvents usable include chloroform, dichloromethane and 1,2,3,4-tetrachlorohexafluorobutane. The case where the 1,2,3,4-tetrachlorohexafluorobutane is used as a solvent, the case has an advantage of no need for separation of products and the solvent in the succeeding fluorination reaction step.

Constitution of a desirable reaction apparatus:
(i) Materials of the reaction apparatus including a reaction vessel and piping include alloys such as stainless steel, inconel, Hastelloy and Monel metal, and glass, polytetrafluoroethylene (PTFE) and perfluoroalkoxyalkanes (PFA), but in the case where the reaction is carried out in the atmospheric pressure, a glass-made, PTFE-made or PFA-made reaction vessel can be used.
(ii) With regard to the reaction of chlorine with 1,3-butadiene, it is preferable that the reaction is carried out by bringing 1,3-butadiene and chlorine gas into contact while the both are fed to the reaction solvent. The amount of the chlorine gas to be introduced is, with respect to the substance amount of 1,3-butadiene, preferably 2.0 to 3.0 mol times and more preferably 2.0 to 2.4 mol times.

Reaction conditions and reaction apparatuses for steps (B) and (C) are as above-mentioned in the "reaction of chlorofluorobutane of the formula (1) with chlorine monofluoride". In the case where steps (A) to (C) are carried out continuously, adoption of the following conditions is desirable in that the yield of 1,2,3,4-tetrachloro-1,1,2,3,4,4-hexafluorobutane being the target substance is improved.
Step (A): temperature (-20 to 20°C), pressure (0 to 0.3 MPaG), other conditions (the amount of Cl₂ to be introduced is, with respect to 1,3-butadiene, preferably 2.0 to 2.4 mol times)
Step (B): temperature (20 to 70°C), pressure (0 to 0.1 MPaG), other conditions (the F₂ gas concentration in the diluted mixed gas is preferably 40 to 70% by volume)
Step (C): temperature (80 to 150°C), pressure (0 to 0.1 MPaG), other conditions (the ClF gas concentration in the diluted mixed gas is preferably 50 to 80% by volume)

### (Other applicable examples)

The 1,2,3,4-tetrachlorobutane can be obtained not only by the reaction of 1,3-butadiene with chlorine, but also by distilling by-products when chloroprene rubber is produced, or by reacting an intermediate, 3,4-dichloro-1-butene, in chloroprene rubber production with chlorine molecules. The production method of the present invention is, since the raw material is easily available relatedly to the chloroprene production, excellent in the mass productivity.

### (Utilization of the inert gas)

The inert gas to be used in the present invention includes nitrogen, helium, neon and argon. The concentration of the inert gas is as described above in each subject item.

### EXAMPLES

The present invention will be described specifically by way of the following Examples, but the scope of the present invention is not limited to the following Examples. The following Examples were carried out according to the following reaction scheme.

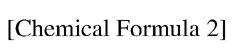

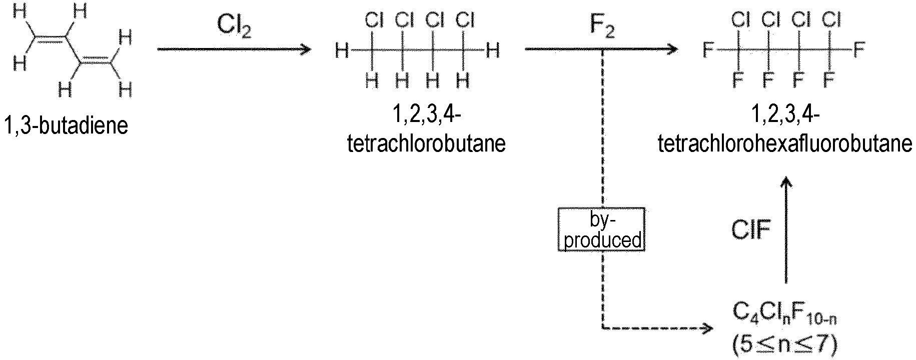

### (Example 1: Chlorination reaction)

In a 4-L SUS-made reactor equipped with a gas introduction port and a gas outlet port connected with a -20°C condenser, 3,267 g of 1,2,3,4-tetrachlorohexafluorobutane (C₄Cl₄F₆) being a final target substance was charged as a reaction solvent, and the reactor temperature was made to be 50°C. Then, from the gas introduction port, 1,3-butadiene gas (C₄H₆) and chlorine gas (Cl₂) were blown into a liquid phase part at flow rates of 11.8 L/h and 31.0 L/h, respectively, and after 9.5 hours, the reaction was terminated. Nitrogen gas was blown into the liquid phase part to purge chlorine gas dissolved in the reaction liquid; thereafter, the reactor was opened and the reaction liquid was taken out. As a result of analysis of the reaction liquid, 1,2,3,4-tetrachlorobutane (C₄H₆Cl₄) was obtained in a yield of 89% (yield amount: 872 g).

### (Example 2: Fluorination reaction)

In a 3-L SUS-made reactor equipped with a gas introduction port and a gas outlet port, 3,000 g of 1,2,3,4-tetrachlorohexafluorobutane (C₄Cl₄F₆) being a final target substance as a solvent and 800 g of 1,2,3,4-tetrachlorobutane (C₄H₆Cl₄) as a raw material were charged, and the reactor temperature was made to be 60°C. Then, from the gas introduction port, a 30-vol% fluorine gas (F₂) diluted with nitrogen gas (N₂) was introduced to a liquid phase part at a flow rate of 60.0 L/h for 20 hours. The reactor temperature was changed to 35°C and a 50-vol% fluorine gas diluted with nitrogen gas was introduced to the liquid phase part for 34 hours at a flow rate of 24.0 L/h, and thereafter, the reaction was terminated. Nitrogen gas was blown into the liquid phase part from the gas introduction port to purge fluorine gas dissolved in the reaction liquid; thereafter, the reactor was opened and the reaction liquid was taken out. As a result of analysis of the reaction liquid, 92% by weight of 1,2,3,4-tetrachlorohexafluorobutane (C₄Cl₄F₆) was contained. Further, as by-products, 6% by weight of 1,1,2,3,4-pentachloropentafluorobutane (C₄Cl₅F₅) was contained.

2,400 g of this reaction liquid was distilled (packed material: Helipack No. 3/HETP: 48 mm, column height: 50 cm, theoretical stage number: 10.4 stages) to thereby obtain 2,086 g of a distillate containing 1,2,3,4-tetrachlorohexafluorobutane (C₄Cl₄F₆) having a purity of 98% by weight or higher, and 242 g of a high-boiling distillate containing 1,1,2,3,4-pentachloropentafluorobutane (C₄Cl₅F₅).

### (Example 3: Cl-F exchange reaction)

In a 200-ml PFA-made reactor equipped with a gas introduction port and a gas outlet port, 200 g of the high-boiling distillate containing 1,1,2,3,4-pentachloropentafluorobutane (C₄Cl₅F₅) obtained by the distillation in Example 2 was charged; a -20°C cooling condenser was connected to the gas outlet port side of the reactor, and the reactor was heated to 130°C. Then, a 50-vol% chlorine monofluoride (ClF) diluted with nitrogen gas (N₂) was introduced to a liquid phase part at a flow rate of 3.6 L/h for 5.5 hours, and thereafter, the reaction was terminated. Nitrogen gas was blown into the liquid phase part from the gas introduction port to purge chlorine monofluoride gas dissolved in the reaction liquid; thereafter, the reactor was opened and 184 g of the reaction liquid was taken out. As a result of analysis of the reaction liquid, the concentration of 1,2,3,4-tetrachlorohexafluorobutane (C₄Cl₄F₆) was 96% by weight.

### (Example 4: Cl-F exchange reaction)

In a 200-ml PFA-made reactor equipped with a gas introduction port and a gas outlet port, 200 g of a 1,1,2,3,4-pentachloropentafluorobutane (C₄Cl₅F₅) having a purity of 97% was charged, and a 0°C collection trap was installed on the gas outlet port side of the reactor. Further, a -20°C cooling condenser was connected to the gas outlet port side of the collection trap, and the reactor was heated to 130°C. Then, a 50-vol% chlorine monofluoride (ClF) diluted with nitrogen gas (N₂) was introduced at a flow rate of 6.0 L/h for 3.5 hours; and thereafter, the reactor temperature was changed to 100°C and the 50-vol% chlorine monofluoride gas was reduced in flow rate to a flow rate of 1.8 L/h, and introduced for 7.0 hours; thereafter, the reaction was terminated. Nitrogen gas was blown in from the gas introduction port to purge chlorine monofluoride gas dissolved in the reaction liquid; thereafter, the reactor was opened and 174 g of the reaction liquid was taken out. As a result of analysis of the reaction liquid, the concentration of 1,2,3,4-tetrachlorohexafluorobutane (C₄Cl₄F₆) was 98%.

### (Example 5: Fluorination reaction)

In a 3-L SUS-made reactor equipped with a gas introduction port and a gas outlet port, 3,000 g of tetrachloromethane (CCl₄) as a solvent and 800 g of 1,2,3,4-tetrachlorobutane (C₄H₆Cl₄) as a raw material were charged, and the reactor temperature was made to be 60°C. Then, from the gas introduction port, a 30-vol% fluorine gas (F₂) diluted with nitrogen gas (N₂) was introduced to a liquid phase part at a flow rate of 60.0 L/h for 20 hours. The reactor temperature was changed to 35°C and a 50-vol% fluorine gas diluted with nitrogen gas was introduced to the liquid phase part at a flow rate of 24.0 L/h for 34 hours, and thereafter, the reaction was terminated. Nitrogen gas was blown into the liquid phase part from the gas introduction port to purge fluorine gas dissolved in the reaction liquid; thereafter, the reactor was opened and the reaction liquid was taken out. The solvent was vacuum distilled out from the reaction liquid, and as a result of analysis of the resultant reaction liquid, 75% by weight of 1,2,3,4-tetrachlorohexafluorobutane (C₄Cl₄F₆) was contained. Further, as by-products, 17% by weight of 1,1,2,3,4-pentachloropentafluorobutane (C₄Cl₅F₅) was contained.

### (Example 6: Cl-F exchange reaction)

In a 200-ml PFA-made reactor equipped with a gas introduction port and a gas outlet port, 200 g of the liquid containing 17% by weight of 1,1,2,3,4-pentachloropentafluorobutane (C₄Cl₅F₅) and 75% by weight of 1,2,3,4-tetrachlorohexafluorobutane obtained by the reaction in Example 5 was charged; a -20°C cooling condenser was connected to the gas outlet port side of the reactor, and the reactor was heated to 130°C. Then, a 50-vol% chlorine monofluoride (ClF) diluted with nitrogen gas (N₂) was introduced at a flow rate of 1.8 L/h for 3.5 hours, and thereafter, the reaction was terminated. Nitrogen gas was blown in from the gas introduction port to purge chlorine monofluoride gas dissolved in the reaction liquid; thereafter, the reactor was opened and 188 g of the reaction liquid was taken out. As a result of analysis of the reaction liquid, the concentration of 1,2,3,4-tetrachlorohexafluorobutane (C₄Cl₄F₆) was 96% by weight. Further, the reaction liquid contained 1,2,3-trichloro-1,1,2,3,4,4,4-heptafluorobutane (C₄Cl₃F₇) and 1,2,4-trichloro-1,1,2,3,3,4,4-heptafluorobutane (C₄Cl₃F₇).

### (Example 7: Cl-F exchange reaction)

1,2,3,4-Tetrachlorohexafluorobutane was synthesized by the same method as in Example 5 and a reaction liquid was taken out. 4,130 g of the reaction liquid was distilled (packed material: Helipack No. 3/HETP: 48 mm, column height: 50 cm, theoretical stage number: 10.4 stages) and tetrachloromethane as a solvent was caused to be distilled. Then, a liquid containing 1,2,3,4-tetrachlorohexafluorobutane (C₄Cl₄F₆) and 1,1,2,3,4-pentachloropentafluorobutane (C₄Cl₅F₅) was caused to be distilled, and 112 g of a residue containing concentrated high-boiling components was obtained. As a result of analysis of this residue, 7% by weight of 1,2,3,4-tetrachlorohexafluorobutane (C₄Cl₄F₆) was contained and 41% by weight of 1,1,2,3,4-pentachloropentafluorobutane (C₄Cl₅F₅) was contained. It was further found that in addition to these, 20% by weight of 1,1,2,3,4,4-hexachloro-1,2,3,4-tetrafluorobutane (C₄Cl₆F₄) was contained. 105 g of the residue was charged in a 100-ml PFA-made reactor equipped with a gas introduction port and a gas outlet port; and a -20°C cooling condenser was connected to the gas outlet port side of the reactor, and the reactor was heated to 130°C. Then, a 50-vol% chlorine monofluoride gas (ClF) diluted with nitrogen gas (N₂) was introduced at a flow rate of 1.8 L/h for 2.0 hours, and thereafter, a part of the reaction liquid was sampled and analyzed. Then, the reaction liquid was again heated and a 50-vol% chlorine monofluoride gas diluted with nitrogen gas was introduced at a flow rate of 1.8 L/h for 2.0 hours, and thereafter, a part of the reaction liquid was similarly sampled and analyzed. Further, the reaction liquid was again heated and a 50-vol% chlorine monofluoride gas diluted with nitrogen gas was introduced at a flow rate of 1.8 L/h for 3.0 hours, and thereafter, the reaction was terminated. Nitrogen gas was blown in from the gas introduction port to purge chlorine monofluoride dissolved in the reaction liquid; and thereafter, the reactor was opened and 93 g of the reaction liquid was taken out. The result of analysis of this reaction liquid is shown in the following Table 1 together with the results in the reaction course.

### [Table 1]

**Table 1**

| Component | % by weight | | | |
|---|---|---|---|---|
| | Raw material | Reaction 2 h | Reaction 4 h | Reaction 7 h |
| 1,2,3,4-Tetrachlorohexafluorobutane | 7 | 14 | 33 | 63 |
| 1,1,2,3,4-Pentachloropentafluorobutane | 41 | 48 | 32 | 1 |
| 1,1,2,3,4,4-Hexachloro-1,2,3,4-tetrafluorobutane | 20 | 3 | 1 | 0 |
| Other compounds | 32 | 35 | 34 | 36 |

### (Example 8: Cl-F exchange reaction)

1,2,3,4-Tetrachlorohexafluorobutane was synthesized by the same method as in Example 2 and a reaction liquid was taken out. The reaction liquid was vacuum distilled (packed material: Helipack No. 3/HETP: 48 mm, column height: 100 cm, theoretical stage number: 20.8 stages) and a liquid containing 1,2,3,4-tetrachlorohexafluorobutane (C₄Cl₄F₆) was caused to be distilled. As a result of analysis of a residue, it was found that 8% by weight of 1,2,3,4-tetrachlorohexafluorobutane and 54% by weight of 1,1,2,3,4-pentachloropentafluorobutane (C₄Cl₅F₅), and 4% by weight of 1,1,2,3,4,4-hexachloro-1,2,3,4-tetrafluorobutane were contained. 250 g of the residue was charged in a 250-ml PFA-made reactor equipped with a gas introduction port and a gas outlet port; and a -20°C cooling condenser was connected to the gas outlet port side of the reactor, and the reactor was heated to 130°C. Then, a 50-vol% chlorine monofluoride gas (ClF) diluted with nitrogen gas (N₂) was introduced at a flow rate of 1.8 L/h for 0.5 hour, and thereafter, a part of the reaction liquid was sampled and analyzed. Then, the reaction liquid was again heated and a 50-vol% chlorine monofluoride gas diluted with nitrogen gas was introduced at a flow rate of 1.8 L/h for 1.0 hour, and thereafter, a part of the reaction liquid was similarly sampled and analyzed. Further, the reaction liquid was again heated and a 50-vol% chlorine monofluoride gas diluted with nitrogen gas was introduced at a flow rate of 1.8 L/h for 11.0 hours, and thereafter, the reaction was terminated. Nitrogen gas was blown in from the gas introduction port to purge chlorine monofluoride dissolved in the reaction liquid; and thereafter, the reactor was opened and 215 g of the reaction liquid was taken out. The result of analysis of this reaction liquid is shown in the following Table 2 together with the results in the reaction course.

### [Table 2]

**Table 2**

| Component | % by weight | | | |
|---|---|---|---|---|
| | Raw material | Reaction 0.5 h | Reaction 1.5 h | Reaction 12.5 h |
| 1,2,3,4-Tetrachlorohexafluorobutane | 8 | 9 | 12 | 60 |
| 1,1,2,3,4-Pentachloropentafluorobutan | 54 | 54 | 52 | 3 |
| 1,1,2,3,4,4-Hexachloro-1,2,3,4-tetrafluorobutane | 4 | 2 | 1 | 0 |
| Other compounds | 34 | 35 | 35 | 37 |

From the above Examples, it was shown that according to the present invention, organic waste liquids (high-boiling components of by-products and the like) generated in the 1,2,3,4-tetrachlorohexafluorobutane production step could be converted to the valuable material, 1,2,3,4-tetrachlorohexafluorobutane. Specifically, in addition to the target substance 1,2,3,4-tetrachlorohexafluorobutane (C₄Cl₄F₆) produced by the fluorination of 1,2,3,4-tetrachlorobutane (C₄H₆Cl₄) with fluorine gas (F₂), 1,1,2,3,4-pentachloropentafluorobutane (C₄Cl₅F₅) and 1,1,2,3,4,4-hexachloro-1,2,3,4-tetrafluorobutane (C₄Cl₆F₄) were by-produced by the fluorination, and could be converted to the target substance 1,2,3,4-tetrachlorohexafluorobutane (C₄Cl₄F₆) by further reacting the 1,1,2,3,4-pentachloropentafluorobutane (C₄Cl₅F₅) and the 1,1,2,3,4,4-hexachloro-1,2,3,4-tetrafluorobutane (C₄Cl₆F₄) with ClF.

### (Example 9: Cl-F exchange reaction)

First and second SUS304-made reactors each having a 25 L-internal volume heating jacket were used. A -25°C cooling condenser was installed to the gas outlet port of the first reactor and the gas outlet port of the cooling condenser was connected to the second reactor gas input port. A -25°C cooling condenser was further installed to the gas outlet port of the second reactor. Then, the liquid outlet port of the second reactor was connected to the liquid input port of the first reactor. Further, the liquid output port of the first reactor was connected to a reaction liquid receiver tank.

29.1 kg of a raw material liquid was charged in each of these reactors. The raw material liquid contained 1% by weight of 1,1,2,3,4,4-hexachlorotetrafluorobutane, 56% by weight of 1,1,2,3,4-pentachloropentafluorobutane and 35% by weight of 1,2,3,4-tetrachlorohexafluorobutane, and was part of cuts of the high-boiling distillate after the distillation obtained by the same method as in Example 2.

The internal liquids of the first reactor and the second reactor were regulated at 105°C, and a mixed gas composed of 97% by volume of chlorine monofluoride gas (ClF) and 3% by volume of hydrogen fluoride gas (HF) was introduced at a flow rate of 11.1 L/h and reaction was initiated. After 80 hours of the reaction, continuous feeding of the raw material liquid from the liquid input port of the second reactor at a flow rate of 0.19 L/h was started. The reaction was carried out in a continuous manner for 120 hours while the ClF gas concentration in the cooling condenser outlet port gas of the succeeding stage of the second reactor was quantitatively analyzed by FT-IR, and as a result of the analysis, the utilization factor of ClF was stabilized at 88%. Further, at this time point, a reaction liquid flowing out from the liquid output port of the first reactor was analyzed, and as a result of the analysis, the composition of the reaction liquid was 0% by weight of 1,1,2,3,4,4-hexachlorotetrafluorobutane, 24% by weight of 1,1,2,3,4-pentachloropentafluorobutane and 66% by weight of 1,2,3,4-tetrachlorohexafluorobutane.

### (Example 10: Cl-F exchange reaction)

The same reaction apparatus as in Example 9 was used, and 29.1 kg of raw material liquid was charged in each of the first reactor and the second reactor. The raw material liquid used was the same as in Example 9.

The internal liquids of the first reactor and the second reactor were regulated at 105°C, and chlorine monofluoride gas (ClF) was introduced at a flow rate of 10.8 L/h and reaction was initiated. After 80 hours of the reaction, continuous feeding of the raw material liquid from the liquid input port of the second reactor at a flow rate of 0.19 L/h was started. The reaction was carried out in a continuous manner for 120 hours while the ClF gas concentration in the cooling condenser outlet port gas of the succeeding stage of the second reactor was quantitatively analyzed by FT-IR, and as a result of the analysis, the utilization factor of ClF was stabilized at 63%. Further, at this time point, a reaction liquid flowing out from the liquid output port of the first reactor was analyzed, and as a result of the analysis, the composition of the reaction liquid was 0% by weight of 1,1,2,3,4,4-hexachlorotetrafluorobutane, 34% by weight of 1,1,2,3,4-pentachloropentafluorobutane and 57% by weight of 1,2,3,4-tetrachlorohexafluorobutane.

## Claims

1. A method for producing 1,2,3,4-tetrachloro-1,1,2,3,4,4-hexafluorobutane, comprising:
step (a) of reacting chlorofluorobutane represented by the following formula (1):
C₄ClₙF₁₀₋ₙ (1)
wherein n is an integer satisfying 5 ≤ n ≤ 7,
with chlorine monofluoride (ClF) to thereby produce 1,2,3,4-tetrachloro-1,1,2,3,4,4-hexafluorobutane.

2. The method according to claim 1, further comprising, before the step (a), step (b) of reacting 1,2,3,4-tetrachlorobutane with fluorine gas (F₂) to thereby produce a reaction liquid comprising chlorofluorobutane represented by the formula (1).

3. The method according to claim 2, wherein in the step (b), 1,2,3,4-tetrachloro-1,1,2,3,4,4-hexafluorobutane is used as a solvent.

4. The method according to any of claims 1 to 3, wherein the chlorofluorobutane represented by the formula (1) comprises 1,1,2,3,4-pentachloro-1,2,3,4,4-pentafluorobutane.

5. A method for producing 1,2,3,4-tetrachloro-1,1,2,3,4,4-hexafluorobutane, comprising:
step (B) of reacting a reaction liquid comprising 1,2,3,4-tetrachlorobutane with fluorine gas (F₂) to thereby produce a reaction liquid comprising 1,2,3,4-tetrachloro-1,1,2,3,4,4-hexafluorobutane and chlorofluorobutane represented by the following formula (1):
C₄ClₙF₁₀₋ₙ (1)
wherein n is an integer satisfying 5 ≤ n ≤ 7; and
step (C) of reacting the reaction liquid obtained by step (B) with chlorine monofluoride (ClF) to thereby convert the chlorofluorobutane represented by the formula (1) in the reaction liquid obtained by step (B) to 1,2,3,4-tetrachloro-1,1,2,3,4,4-hexafluorobutane.

6. The method according to claim 5, wherein the chlorofluorobutane represented by the formula (1) comprises 1,1,2,3,4-pentachloro-1,2,3,4,4-pentafluorobutane.

7. The method according to claim 5 or 6, further comprising step (A) of reacting 1,3-butadiene with chlorine gas (Cl₂) to thereby produce a reaction liquid comprising 1,2,3,4-tetrachlorobutane, wherein the reaction liquid obtained by step (A) is used as a raw material of step (B).

8. The method according to any of claims 1 to 3, wherein in the step (a), the reaction is carried out in the presence of hydrogen fluoride (HF).

9. The method according to claim 4, wherein in the step (a), the reaction is carried out in the presence of hydrogen fluoride (HF).

10. The method according to claim 5 or 6, wherein in the step (C), the reaction is carried out in the presence of hydrogen fluoride (HF).

11. The method according to claim 7, wherein in the step (C), the reaction is carried out in the presence of hydrogen fluoride (HF).
